# EUROPÄISCHE PATENTSCHRIFT

(11) **EP 3 066 205 B1**
(45) Veröffentlichungstag und Bekanntmachung des Hinweises auf die Patenterteilung: **25.12.2019**
(21) Anmeldenummer: 14798780.4
(22) Anmeldetag: 10.11.2014
(51) Int. Cl.: C12P 5/02, C12P 3/00

(54) **VERFAHREN ZUR HERSTELLUNG VON BIOGAS ENTHALTEND EINE VERRINGERUNG DER AMMONIUMKONZENTRATION DURCH ANAMMOX**
PROCESS FOR PRODUCING BIOGAS COMPRISING A REDUCTION OF AMMONIUM CONCENTRATION BY ANAMMOX
PROCÉDÉ DE PRODUCTION DE BIOGAZ COMPRENANT UNE RÉDUCTION DE LA CONCENTRATION EN AMMONIUM EN UTILISANT L'ANAMMOX

(30) Priorität: 10.11.2013 DE 102013018833
(43) Veröffentlichungstag der Anmeldung: 14.09.2016
(73) Patentinhaber: Ellmann Engineering (EE) GmbH, 06636 Laucha (DE)
(72) Erfinder: ELLMANN, Reik, 06636 Laucha an der Unstrtut (DE)
(74) Vertreter: K & H Bonapat Patentanwälte Koch · von Behren & Partner mbB
(86) Internationale Anmeldenummer: PCT/EP2014/074206
(87) Internationale Veröffentlichungsnummer: WO 2015/067813

(56) Entgegenhaltungen:
- CA-A1- 2 860 583
- SIEGRIST, H. ET AL.: "Anammox brings WWTP closer to energy autarky due to increased biogas production and reduced aeration energy for N-removal", WATER SCIENCE & TECHNOLOGY, Bd. 57, Nr. 3, 2008, Seiten 383-388, XP002736407,
- MAGRÍ, A. ET AL.: "Feasibility and interest of the anammox process as treatment alternative for anaerobic digester supernatants in manure processing - An overview", JOURNAL OF ENVIRONMENTAL MANAGEMENT, Bd. 131, 25. Oktober 2013 (2013-10-25), Seiten 170-184, XP028780597,
- DATABASE BIOSIS [Online] BIOSCIENCES INFORMATION SERVICE; 2010, HU, A. ET AL.: "Hydroxylamine conversion by anammox enrichment", XP002736409, Database accession no. 2010:268564
- KIMURA, Y. ET AL.: "Effects of inorganic carbon limitation on anaerobic ammonium oxidation(anammox) activity", BIORESOURCE TECHNOLOGY, Bd. 102, Nr. 6, 1. Januar 2011 (2011-01-01), Seiten 4390-4394, XP002736428,

## Beschreibung

Die Erfindung betrifft ein Verfahren, bei dem der aus einem Fermenterreaktor stammende Reststoff als Anmaischsubstrat für feste oder feststoffhaltige organische Biomasse zur Biogasgenerierung aufbereitet wird.

Zu den Stoffen, die insbesondere zur Biogasgenerierung einem anaeroben Fermenterreaktor zugeführt werden, zählen Luzerne, Hühnermist oder Hühnergülle, proteinreiche Substanzen wie Schlachtabfälle oder Molkereiabwässer oder diesbezügliche allgemeine Abfälle. Dabei ist es zum Abbau fester und flüssiger organischer Biomasse bekannt, ein Rohgemenge - ggf. mit vorgeschalteter mechanischen Behandlung (bspw. Sandfang) - zu versäuern und einer Fermentation, d.h. einer Vergärung zur Biogasgenerierung zuzuführen. Bei dieser Methanisierung fällt neben der Produktion des eigentlichen Biogases auch ein Reststoffanteil als Gärrest an, der als Kompost ausgetragen oder als Düngerzusatz weiterverarbeitet wird.

Bei der Verarbeitung des Gärrestes zu einem Düngerrohstoff kann der Gärrest nachfolgend einer mechanischen Aufbereitung mit einer Trennung zur Eindickung in zwei Teilströmen weitergeführt werden. Während der erste Teilstrom einer Trocknung zur Produktion eines trockenen Düngers unterzogen wird, durchläuft der zweite Teilstrom des nach der Verpressung verbleibenden Flüssiganteils oder Dünnlaufs im Stand der Technik eine Eindampfung mit einem Trockensubstanzanteil von ca. 15%. Beide Rohstoffe können, wenn diese hinsichtlich ihrer Nährstoffe hochwertig sind, landwirtschaftlich verwertet werden, sie andernfalls entsorgt werden müssen.

Im Stand der Technik besteht das Problem, dass sich nicht jede Ausgangs-Biomasse gleichermaßen zur Fermentation eignet. Häufig bleiben nicht fermentierbare Rückstände übrig, die entsorgt oder gesondert verwertet werden müssen. Unzureichend fermentierbar sind insbesondere Rückstände, die Lignin oder Ähnliches enthalten.

Auch ist es problematisch, dass bei einigen Ausgangs-Biomassen für Biogasanlagen hohe Konzentrationen an Stickstoff in Form von Ammoniumverbindungen vorliegen. Ammonium führt bei erhöhten Konzentrationen nachteilhaft zu einer Wachstumshemmung der für die Methanogenese erforderlichen anaeroben Bakterien und damit zu einer Verminderung der Biogasproduktion oder gar zur völligen Blockade des biologischen Systems. Überdies ist der relative Methananteil im Biogas dann durch einen hohen Ammoniakgehalt reduziert. Bei der Verbrennung eines solchen Stickstoffverbindungen enthaltenden Gases zur Energiegewinnung entstehen überdies umweltbelastende Stickoxide, die es zu vermeiden gilt.

Das Problem eines zu hohen Stickstoffanteils bei der Klärung von Biomasse kennt man bereits aus Abwasseranlagen für kommunale Abwässer. Hier wird zur Reduzierung des Stickstoffgehaltes u.a. das sog. Anammox-Verfahren eingesetzt, bei dem der Stoffwechsel des Bakteriums *Brocadia anammoxidans* genutzt wird, um Ammonium unter anaeroben Bedingungen mit Nitrit zu molekularem Stickstoff umzusetzen.

In dem Dokument von KIMURA, Y. ET AL., "Effects of inorganic carbon limitation on anaerobic ammonium oxidation (anammox) activity", BIORESOURCE TECHNOLOGY, (20110101), Vol. 102, Nr. 6, wurde eine Beziehung zwischen einer Zufluss-IC-Konzentration (IC: anorganischer Kohlenstoff) und einer Anammox-Aktivität unter Verwendung der Michaelis-Menten-Kinetik analysiert. Es wurde bestimmt, dass der ersichtliche Parameter Kₘ 1,2 mg C/L betrug. Die Aktivität konnte durch Zugabe von IC zu dem Zulauf wiedergewonnen werden. Das Verbrauchsverhältnis von IC zu Ammonium war nicht konstant und hing hauptsächlich vom Zuflussverhältnis des IC zu Ammoniumkonzentrationen (inf. IC/inf. NH₄-N) ab. Die Ergebnisse zeigten, dass ein inf. IC/inf. NH₄-N-Verhältnis von 0,2 in einem Anammox-Reaktor für das Anammox-Verfahren unter Verwendung von Gel-Würfeln ideal war.

In dem Dokument von HU, A. ET AL., "Hydroxylamine conversion by anammox enrichment", DATABASE BIOSIS, BIOSCIENCES INFORMATION SERVICE, Database accession no. 2010:268564, wurde festgestellt, dass eine Anammox-Anreicherung in der Lage war, gleichzeitig Hydroxylamin und Nitrit umzuwandeln, und eine Umwandlungsrate für Hydroxylamin aufwies, die höher war als eine Umwandlungsrate für Nitrit.

Das Dokument von MAGRÍ, A. ET AL., "Feasibility and interest of the anammox process as treatment alternative for anaerobic digester supernatants in manure processing - An overview", JOURNAL OF ENVIRONMENTAL MANAGEMENT, (20131025), Vol. 131, bietet einen Überblick über veröffentlichte Studien über ANR (autotrophische Stickstoffentfernung). Spezielle Fragen in Bezug auf die Anwendbarkeit des Verfahrens zur Behandlung von Düngergärresten werden diskutiert. Der Energiebedarf von ANR wird mit jenen anderer technologischer Alternativen verglichen, die auf die Gewinnung von Stickstoff aus Faulbehälterüberständen abzielen. Es wurde gezeigt, dass die Ergebnisse der Beurteilung von der Zusammensetzung des Überstands abhängen. In dieser Hinsicht erwies sich das PN-Anammox-Verfahren (PN: partielle Nitritation) als konkurrenzfähiger als andere Alternativen, insbesondere in Konzentrationen von bis zu 2 kg NH₄⁺-N m⁻³.

In CA 2 860 583 wird ein Verfahren einer Behandlung eines zu behandelnden Abwassers beschrieben zum Zweck der Senkung seines Phosphatgehaltes. Das Verfahren umfasst einen Schritt einer anoxischen biologischen Behandlung des zu behandelnden Abwassers, die ein erstes Abwasser erzeugt, einen Schritt einer aeroben biologischen Behandlung der ersten Abwassers, die ein zweites Abwasser erzeugt, einen Schritt der Rückführung von zumindest einem Teil des zweiten Abwassers zu dem Einlass der anoxischen biologischen Behandlung, einen Schritt einer Flüssigkeit/Festkörper-Trennung von zumindest einem Teil des zweiten Abwassers, der ein behandeltes Abwasser und ein erstes dichteres Abwasser erzeugt, einen Schritt zur Bildung von flüchtigen Fettsäuren umfassend eine Nasswärmebehandlung bei einer Temperatur von 100 °C bis 350 °C für eine Verweilzeit von zwischen 10 und 180 Minuten von zumindest einem Teil des ersten dichteren Abwassers, einen Schritt einer anaeroben biologischen Behandlung von zumindest einem Teil des Abwassers, das von dem Schritt der Nasswärmebehandlung herrührt, und einen Schritt der Rückführung von zumindest einem Teil des Abwassers, das von dem Schritt der anaeroben biologischen Behandlung herrührt, zu dem Einlass des Schrittes der anoxischen biologischen Behandlung.

In dem Dokument von SIEGRIST, H. ET AL., "Anammox brings WWTP closer to energy autarky due to increased biogas production and reduced aeration energy for N-removal", WATER SCIENCE & TECHNOLOGY, (2008), Vol. 57, Nr. 3, ist beschrieben, dass erst vor kurzem eine ammoniakreiche Fermentationsflüssigkeit (15-20% der Einlass-Ammoniakbelastung) mit einem sehr ökonomischen autotrophischen Nitritierungs-/Anammox-Verfahren behandelt werden konnte, das die Hälfte der Belüftungsenergie und keine organische Kohlenstoffquelle im Vergleich zur Nitrifikation und heterotrophen Denitrifikation benötigt. Mit der Einführung dieser neuen innovativen Faulbehälterflüssigkeitsbehandlung kehrt die Situation zurück, die es uns ermöglicht, die hydraulische Retentionszeit des Primärklärers von kommunalen Kläranlagen zu erhöhen, um die Biogasproduktion zu verbessern und die Belüftungsenergie für die Entfernung des biochemischen Sauerstoffbedarfs und Nitrifizierung bei ähnlicher Gesamt-Stickstoffentfernung zu reduzieren.

Aufgabe der vorliegenden Erfindung ist es, ein Verfahren der eingangs genannten Art anzugeben, bei dem der Gärrest eines Fermenters so aufbereitet wird, dass er danach geeignet zur weiteren Energiegewinnung als Additiv für frische Biomasse zur erneuten Biogasgenerierung verwertet werden kann.

Diese Aufgabe wird grundlegend durch das im Anspruch 1 beschriebene Verfahren gelöst:
Verfahren zur Aufbereitung eines aus einem Fermenterreaktor stammenden Reststoffes in Form von Gärrest als Anmaischsubstrat für feste oder feststoffhaltige organische Biomasse für eine Biogasgenerierung, bei dem
- eine Fest-Flüssigtrennung des Reststoffes erfolgt,
- der flüssige Anteil als Dünnlauf einem isolierten Reaktionsbehälter zugeführt wird, in dem der Dünnlauf einer anaeroben Ammonium-Oxidation unter Nutzung von Anammox-Organismen zur Reduzierung der Ammoniumkonzentration im Submersverfahren unterzogen wird, wobei die Zönose des Reaktionsbehälters auf einer Temperatur von zwischen 35 bis 45 °C gehalten wird,
- und nach Ablauf einer Verweildauer im Reaktionsbehälter das stickstoffabgereicherte Substrat als Prozesswasser einer für die Biogasfermentation bestimmten Biomasse zugemischt und zusammen mit dieser in eine Hydrolyse als die erste Stufe eines Biogasprozesses geführt wird, wobei die Anammox-Organismen als autotrophe Organismen ein vielfältig in der Hydrolyse entstehendes Kohlenstoffdioxid in Form von wasserlöslichem Hydrogencarbonat als Wachstumssubstrat nutzen und damit einen relativen Kohlenstoffdioxidgehalt im Hydrolysegas und im finalen Biogas senken, und
- ein Vorquellen der Biomasse stattfindet, wenn das stickstoffarme Prozesswasser bei der Temperatur von zwischen 35 bis 45 °C als Anmaischwasser verwendet wird.

Vorteilhafte Ausführungsformen gehen aus den nachgeordneten Unteransprüchen hervor.

Kerngedanke der Erfindung ist es, den Reststoff der Biogasanlage so zu modifizieren, dass er nach Aufbereitung in die Biogasproduktion rückgeführt werden kann, bzw. als Prozesswasser einer zur Biogasgewinnung bestimmten Biomasse zugemischt werden kann und auf diese geeignet einwirkt. Besagte Aufbereitung betrifft dabei eine für die Biogasgenerierung erforderliche Reduzierung des Ammoniumgehaltes im Ausgangssubstrat. Dadurch erzielt die Erfindung eine Minimierung der Ammoniakfreisetzung in der eigentlichen Hydrolysestufe.

Erfindungsgemäß wird zunächst der aus der Biogasgenerierung stammende Reststoff, d.h. der Gärrest einer Fest-Flüssigtrennung unterzogen. Die hieraus resultierende wässrige Phase, der sog. Dünnlauf wird sodann einer anoxischen biotechnologischen Stufe zugeführt. Diese umfasst einen Biomassewachstumsreaktor, in der spezielle, stickstoffbildende Mikroorganismen gebildet werden. Zu diesen gehören *Brocadia anammoxidans.* In dem Reaktor kommt es durch einen hohen Gehalt an diesen speziellen Mikroorganismen zur wesentlichen Reduzierung des Ammoniumstickstoffs und des Gesamtstickstoffs. Letzteres erfolgt dadurch, dass Ammonium mit Nitrit unter anaeroben Bedingungen zu Stickstoff umgesetzt wird.

Als erfindungswesentlich wurde hierbei erkannt, dass die zur Spaltung des Stickstoffs genutzten Mikroorganismen autotrophe Organismen sind, die, wenn das stickstoffabgereicherte Substrat mitsamt der herangewachsenen Mikroorganismen als Prozesswasser unter eventueller Mischung mit neuer Biomasse rezirkuliert und dem Hydrolyseverfahren erneut zugeführt wird, das vielfältig in der Hydrolyse entstehende Kohlenstoffdioxid als Wachstumssubstrat nutzen und damit den relativen Kohlenstoffdioxidgehalt im Hydrolysegas und im finalen Biogas senken.

Stickstoffspaltende Mikroorganismen sind autotrophe Mikroorganismen, d.h. ihre Kohlenstoffquelle ist Kohlenstoffdioxid bzw. die entsprechende wasserlösliche Form Hydrogencarbonat. In der flüssigen Phase aller Biogasanlagen ist Hydrogencarbonat in hohen Konzentrationen vorhanden. Es wirkt als Puffersystem und stabilisiert so den pH-Wert. Die Konzentration an Hydrogencarbonat wird als TAC (Total Anorganic Carbon) gemessen und kontrolliert. Durch die hohe Verfügbarkeit dieses Kohlenstoffanteils gerade bei Biogasanlagen kommt es zu einem verstärkten Wachstum der stickstoffbildenden Mikroorganismen und dadurch zu ihrer Anreicherung. Die vorliegende Erfindung arbeitet in einem TAC Wertebereich von 5000,- bis 30000,- mg HCO₃/l.

Als erfindungswesentlich wurde ferner festgestellt, dass sich auf das Wachstum außerdem eine hohe Temperatur begünstigend auswirkt, da sie nicht nur die Substrataufnahme fördert, sondern auch das Wachstum von Konkurrenten behindert. Deshalb wird in dem separaten Anzucht-Reaktionsbehälter die Zönose bei Temperaturn um 40 °C, erfindungsgemäß zwischen 35 bis 45 °C gehalten. Das Wachstum der Anammoxbakterien selbst ist bei höheren Temperaturen beschleunigt. Jedoch sinkt mit steigenden Temperaturen die Kohlenstoffdioxidlöslichkeit und damit dessen Verfügbarkeit für die Mikroorganismen im Wasser. Erfindungsgemäß musste somit eine optimale Temperatur im mesophilen Bereich gefunden werden, die mit angegebenem Bereich von zwischen 35 bis 45 °C erfindungsgemäß in Versuchen konstatiert werden konnte.

Beim folgenden Prozessschritt, dem Anmaischen von neuer Biomasse mit dem aufbereiteten Gärrest-Produkt, wirkt sich diese hohe Temperatur der Anmaischsuspension beschleunigend auf das Aufquellen trockener, kohlenhydratreicher Substrate aus. Damit wirkt das aufbereitete Gärrest-Produkt auch auf solche Biomasse positiv ein, die sich originär für eine Biogasgenerierung nicht so eignen würde. Schließlich kommt es durch das spezielle Anmaischen mit dem aus der Aufbereitung des Gärrestes erhaltenen Prozesswasser zu einer Beschleunigung des Hydrolyseprozesses. Mit anderen Worten: Wird das stickstoffarme Prozesswasser als Anmaischwasser verwendet, kann durch Vorquellen der Biomasse eine schnellere Hydrolyse stattfinden. Damit wird der Gesamtprozess der Biogasbildung beschleunigt.

Für den Energiegewinnungsprozess der Mikroorganismen als Voraussetzung für ihr Wachstum ist Sauerstoff zur Umwandlung von Ammonium- über Hydroxylamin zu Nitritstickstoff notwendig. Aus diesem Grund ist vorteilhaft vorgesehen, den Aufzucht-Reaktor zu belüften. Allerdings führt eine hohe Sauerstoffzufuhr zur Weiteroxidation des Nitrit- zum Nitratstickstoff. Deshalb muss der Sauerstoffeintrag begrenzt, kontrolliert und geregelt gestaltet werden. Da der Sauerstoffgehalt sehr niedrig und damit außerhalb des Messbereichs üblicher Sauerstoffelektroden ist, wird die Regelung in vorteilhafter Weise über das Redoxpotenzial vollzogen. Dabei werden substratspezifische Werte zur Regelung genutzt, die vom Gehalt reduzierender Substanzen im Gärrest bestimmt werden.

Die Erfindung erzielt unter Nutzung des Kohlenstoffanteils des Gärrestes damit den Vorteil einer Erhöhung der verfügbaren Konzentration an Anammoxbakterien, durch deren Wirkung eine Reduzierung des Ammonium- und Gesamtstickstoffgehaltes schneller und effektiver möglich und damit eine Wiederverwendung des nunmehr stickstoffarmen Wassers als Anmaischwasser möglich ist. Dabei sind Zulaufkonzentrationen von Ammonium von bis zu 7 g/l verwertbar.

Gemäß einer vorteilhaften Ausführungsform wird die Belüftung des Reaktors zur Nitritbildung und/oder die Dauer und die Intensität einer Umwälzung der Zönose im Reaktor gesteuert. Für die Umwälzung werden mechanische Rührwerke, hydraulische Rührsysteme oder Gaseinpressungen eingesetzt.

Als vorteilhaft hat sich auch gezeigt, dass der Gehalt an Hydroxylamin in der Behälter-Zönose ermittelt wird, um daraus eine Konzentration der Anammox-Organismen abzuleiten.

Die Verweildauer der Zönose im Reaktorbehälter beträgt vorteilhaft zwischen 24 Stunden und 6 Tagen.

Bei diskontinuierlicher Entnahme des stickstoffabgereicherten Substrats aus dem Reaktionsbehälter werden vorteilhaft von diesem maximal nur 40 bis 80% pro Charge entnommen, um das Wachstum der Organismen in einem stabilen Gleichgewicht zu halten.

Zur vorteilhaften Steuerung des Prozesses wird der pH Wert, die Leitfähigkeit und das Redoxpotential des Reaktorinhaltes kontinuierlich gemessen und aufgezeichnet.

## Patentansprüche

1. Verfahren zur Aufbereitung eines aus einem Fermenterreaktor stammenden Reststoffes in Form von Gärrest als Anmaischsubstrat für feste oder feststoffhaltige organische Biomasse für eine Biogasgenerierung, bei dem
- eine Fest-Flüssigtrennung des Reststoffes erfolgt,
- der flüssige Anteil als Dünnlauf einem isolierten Reaktionsbehälter zugeführt wird, in dem der Dünnlauf einer anaeroben Ammonium-Oxidation unter Nutzung von Anammox-Organismen zur Reduzierung der Ammoniumkonzentration im Submersverfahren unterzogen wird, wobei die Zönose des Reaktionsbehälters auf einer Temperatur von zwischen 35 bis 45 °C gehalten wird,
- und nach Ablauf einer Verweildauer im Reaktionsbehälter das stickstoffabgereicherte Substrat als Prozesswasser einer für die Biogasfermentation bestimmten Biomasse zugemischt und zusammen mit dieser in eine Hydrolyse als die erste Stufe eines Biogasprozesses geführt wird, wobei die Anammox-Organismen als autotrophe Organismen ein vielfältig in der Hydrolyse entstehendes Kohlenstoffdioxid in Form von wasserlöslichem Hydrogencarbonat als Wachstumssubstrat nutzen und damit einen relativen Kohlenstoffdioxidgehalt im Hydrolysegas und im finalen Biogas senken, und
- ein Vorquellen der Biomasse stattfindet, wenn das stickstoffarme Prozesswasser bei der Temperatur von zwischen 35 bis 45 °C als Anmaischwasser verwendet wird.

2. Verfahren nach Anspruch 1,
**dadurch gekennzeichnet, dass** zur Produktion von Nitrit ein Sauerstoffeintrag in den Reaktionsbehälter erfolgt, wobei ein Sauerstoffeintrag geregelt wird und eine Regelung über ein Redoxpotenzial vollzogen wird, wobei substratspezifische Werte zur Regelung genutzt werden, die von einem Gehalt reduzierender Substanzen in dem aus dem Fermenterreaktor stammenden Reststoff bestimmt werden.

3. Verfahren nach einem der vorangehenden Ansprüche,
**dadurch gekennzeichnet, dass** eine gesteuerte Durchmischung der Behälter-Zönose erfolgt, wobei eine Dauer und Intensität einer Umwälzung der Zönose in dem Behälter gesteuert wird und für die Umwälzung mechanische Rührwerke, hydraulische Rührsysteme oder Gaseinpressungen eingesetzt werden.

4. Verfahren nach einem der vorangehenden Ansprüche,
**dadurch gekennzeichnet, dass** der Gehalt an Hydroxylamin in der Behälter-Zönose ermittelt wird, aus dem eine Konzentration der Anammox-Organismen abgeleitet wird.

5. Verfahren nach einem der vorangehenden Ansprüche,
**dadurch gekennzeichnet, dass** der anorganische Kohlenstoffanteil der Behälter-Zönose als TAC (total anorganic carbon) in einem Bereich von 5000 bis 30000 mg HCO₃ pro Liter gehalten wird.

6. Verfahren nach einem der vorangehenden Ansprüche,
**dadurch gekennzeichnet, dass** die Verweildauer im Reaktionsbehälter zwischen 24 h und 6 Tagen beträgt.

7. Verfahren nach einem der vorangehenden Ansprüche,
**dadurch gekennzeichnet, dass** bei diskontinuierlicher Entnahme des stickstoffabgereicherten Substrats aus dem Reaktionsbehälter von diesem maximal 40 bis 80% pro Charge entnommen werden, um ein Wachstum der Anammox-Organismen in einem stabilen Gleichgewicht zu halten.

8. Verfahren nach einem der vorangehenden Ansprüche,
**dadurch gekennzeichnet, dass** unter Nutzung eines Kohlenstoffanteils des aus dem Fermenterreaktor stammenden Reststoffes eine verfügbare Konzentration an Anammox-Organismen erhöht wird, durch deren Wirkung eine Reduzierung eines Ammonium- und Gesamtstickstoffgehaltes schneller und effektiver möglich und damit eine Wiederverwendung des nunmehr stickstoffarmen Prozesswassers als Anmaischwasser möglich ist.

9. Verfahren nach dem vorangehenden Anspruch,
**dadurch gekennzeichnet, dass** Zulaufkonzentrationen von Ammonium von bis zu 7 g/l verwertet werden.

10. Verfahren nach einem der vorangehenden Ansprüche,
**dadurch gekennzeichnet, dass** zur Steuerung des Prozesses der Zönose des Reaktionsbehälters ein pH-Wert, eine Leitfähigkeit und ein Redoxpotenzial eines Reaktionsbehälterinhaltes kontinuierlich gemessen und aufgezeichnet werden.

## Claims

1. Method for preparing a residue originating from a fermenter reactor, in the form of digestate, as mashing substrate for solid or solids-containing organic biomass for biomass generation, in which
solid-liquid separation of the residue is performed,
the liquid portion is supplied as a thin-running substance to an isolated reaction container, in which the thin-running substance is subjected to anaerobic ammonium oxidation using anammox organisms to reduce the ammonium concentration in a submersion method, the coenosis of the reaction container being kept at a temperature of between 35 and 45 °C, and, after a retention time in the reaction container has elapsed, the nitrogen-depleted substrate is mixed as process water into a biomass intended for the biogas fermentation, and guided together therewith to a hydrolysis as the first step of a biogas process, the anammox organisms, as autotrophic organisms, using a carbon dioxide, which occurs diversely in the hydrolysis, in the form of water-soluble hydrogen carbonate as a growth substrate, and thus decreasing a relative carbon dioxide content in the hydrolysis gas and in the final biogas, and
advance swelling of the biomass taking place when the low-nitrogen process water is used as mashing water at the temperature of between 35 and 45 °C.

2. Method according to claim 1,
**characterised in that**, for producing nitrite, oxygen is introduced into the reaction container, an oxygen intake being regulated and regulation being carried out by way of a redox potential, substrate-specific values, which are determined from a content of reducing substances in the residue originating from the fermenter reactor, being used for the regulation.

3. Method according to any of the preceding claims,
**characterised in that** the container coenosis is thoroughly mixed in a controlled manner, a duration and intensity of a circulation of the coenosis in the container being controlled, and mechanical stirring units, hydraulic stirring systems or gas injections being used for the circulation.

4. Method according to any of the preceding claims,
**characterised in that** the hydroxylamine content in the container coenosis is determined, a concentration of the anammox organisms being derived from said content.

5. Method according to any of the preceding claims,
**characterised in that** the inorganic carbon portion of the container coenosis, as the TIC (total inorganic carbon), is kept in a range of 5000 to 30000 mg HCO₃ per litre.

6. Method according to any of the preceding claims,
**characterised in that** the retention time in the reaction container is between 24 h and 6 days.

7. Method according to any of the preceding claims,
**characterised in that**, when the nitrogen-depleted substrate is withdrawn from the reaction container, at most 40 to 80 % thereof is removed per batch, so as to keep growth of the anammox organisms in stable equilibrium.

8. Method according to any of the preceding claims,
**characterised in that**, using a carbon portion of the residue originating from the fermenter reactor, an available concentration of annamox organisms is increased, the effect of said organisms making a decrease in an ammonium and total nitrogen content more rapid and more effective, and it thus being possible to reuse the now low-nitrogen process water as mashing water.

9. Method according to the preceding claim,
**characterised in that** feed concentrations of ammonium of up to 7 g/l are utilised.

10. Method according to any of the preceding claims,
**characterised in that**, to control the process of the coenosis of the reaction container, a pH, a conductivity and a redox potential of a reaction container content are measured and displayed continuously.

## Revendications

1. Procédé de traitement d'un résidu provenant d'un réacteur de fermentation sous forme de résidu gazeux en substrat d'empâtage pour une biomasse organique solide ou contenant des matières solides destinée à la production de biogaz, dans lequel
il se produit une séparation solide-liquide du résidu,
la partie liquide est amenée sous forme de fraction liquide vers un récipient réactionnel isolé, en ce que la fraction liquide est soumise à une oxydation anaérobie de l'ammonium moyennant l'utilisation d'organismes anammox pour la réduction de la concentration en ammonium dans un procédé de submersion, dans lequel la cénose du récipient réactionnel est maintenue à une température entre 35 et 45 °C, et, après l'écoulement d'une durée de séjour dans le récipient réactionnel, le substrat appauvri en azote est mélangé sous forme d'eau de procédé à une biomasse destinée à la fermentation du biogaz et est soumis conjointement avec celle-ci à une hydrolyse servant de premier stade pour un processus de biogaz, dans lequel les organismes anammox sous forme d'organismes autotrophes utilisent du dioxyde de carbone créé de manière multiple dans l'hydrolyse sous forme d'hydrogénocarbonate soluble dans l'eau en tant que substrat de croissance et par conséquent diminuent une teneur en dioxyde de carbone relative dans le gaz d'hydrolyse et dans le biogaz final, et
il se produit un pré-gonflement de la biomasse lorsque l'eau de procédé pauvre en azote est employée en tant qu'eau d'empâtage à une température entre 35 et 45 °C.

2. Procédé selon la revendication 1,
**caractérisé en ce que**, pour la production de nitrite, il y a un apport d'oxygène dans le récipient réactionnel, dans lequel l'apport d'oxygène est régulé et qu'il y a une régulation par le biais d'un potentiel rédox, dans lequel des grandeurs spécifiques au substrat sont employées pour la régulation, qui sont déterminées par une teneur en substances de réduction d'un résidu provenant du réacteur de fermentation.

3. Procédé selon l'une des revendications précédentes,
**caractérisé en ce qu'**il y a un mélange contrôlé de la cénose du récipient, dans lequel une durée et une intensité d'une circulation de la cénose est contrôlée dans le récipient, et des agitateurs mécaniques, des systèmes d'agitation hydrauliques ou des injections de gaz sous pression sont mis en œuvre pour la circulation.

4. Procédé selon l'une des revendications précédentes,
**caractérisé en ce que** la teneur en hydroxylamine dans la cénose du récipient est déterminée, à partir de laquelle une concentration des organismes anammox est déduite.

5. Procédé selon l'une des revendications précédentes,
**caractérisé en ce que** la proportion de carbone inorganique de la cénose du récipient sous forme de TAC (total anorganic carbon, carbone inorganique total) est maintenue dans une plage de 5000 à 30000 mg de HC0₃ par litre.

6. Procédé selon l'une des revendications précédentes,
**caractérisé en ce que** la durée de séjour dans le récipient réactionnel est entre 24 heures et 6 jours.

7. Procédé selon l'une des revendications précédentes,
**caractérisé en ce que**, pour un prélèvement discontinu du substrat appauvri en azote à partir du récipient réactionnel, au maximum de 40 à 80 % par charge sont prélevés afin de maintenir la croissance des organismes anammox à un niveau stable.

8. Procédé selon l'une des revendications précédentes,
**caractérisé en ce que**, moyennant une utilisation d'une partie du carbone du résidu provenant du réacteur de fermentation, une concentration disponible d'organismes anammox est augmentée, dont l'effet rend une réduction plus rapide et efficace d'une teneur en ammonium et en azote total possible et ainsi une réutilisation de l'eau de procédé à présent pauvre en azote en tant qu'eau d'empâtage.

9. Procédé selon la revendication précédente,
**caractérisé en ce que** des concentrations d'apport d'ammonium jusqu'à 7 g/l sont employées.

10. Procédé selon l'une des revendications précédentes,
**caractérisé en ce que**, pour le contrôle du processus de la cénose du récipient réactionnel, la valeur du pH, la conductibilité et le potentiel rédox du contenu du récipient réactionnel sont continuellement mesurés et enregistrés sur un graphe.
